# EUROPÄISCHE PATENTSCHRIFT

(11) **EP 1 316 326 B1**
(45) Veröffentlichungstag und Bekanntmachung des Hinweises auf die Patenterteilung: **02.05.2007**
(21) Anmeldenummer: 01128260.5
(22) Anmeldetag: 28.11.2001
(51) Int. Cl.: A61M 25/10

(54) **Katheter-Ballon**
Catheter balloon
Ballonnet de cathéter

(43) Veröffentlichungstag der Anmeldung: 04.06.2003
(73) Patentinhaber: Abbott Laboratories Vascular Enterprises Limited, Dublin 2 (IE)
(72) Erfinder: Quint, Bodo, 72108 Rottenburg/Seebronn (DE)
(74) Vertreter: Schmitz, Hans-Werner

(56) Entgegenhaltungen:
- EP-A- 0 783 897
- US-A- 5 041 125
- US-A- 5 853 389
- US-A- 6 013 055

## Beschreibung

Die Erfindung betrifft einen Katheter-Ballon gemäß Anspruch 1.

Ein derartiger Ballon ist aus der US-5,853,389 bzw. der US-A-5,041,125 bekannt.

Der Ballon weist einen kollabierbaren Ballonkörper auf, der auf einem Katheterschaft so aufzubringen ist, dass er vor seiner Expansion möglichst wenig Platz einnimmt. Hierzu wird der Katheter-Ballon, so wie seine sich verjüngenden Endbereiche, mit Faltungen versehen. Neben der Eigenschaft, dass der Katheter-Ballon im Bereich des Ballons ein möglichst geringes Profil aufweisen soll, um somit beispielsweise einfach durch eine Stenose geführt werden zu können, haben im Rahmen der Erfindung durchgeführte Untersuchungen ergeben, dass bei einem Vorsehen einer Faltung auf dem Ballonkörper und den verjüngten Enden das Verhalten beim Einfalten verbesserungsbedürftig ist. Insbesondere hat sich hierbei gezeigt, dass das Rückfaltvermögen des Ballons ein Problem darstellt. Denn der Ballon verliert durch den Zyklus von Inflatieren und Deflatieren und die damit einhergehende mechanische Belastung sein Memory-Verhalten an die ursprüngliche Faltung. Dies kann beispielsweise zu erheblichen Schwierigkeiten führen, wenn der Ballon durch zwei hintereinander liegende Stenosen geführt wird. Wird der Ballon nach dem Aufweiten der ersten Stenose deflatiert und ergibt sich durch den Verlust des Memory-Verhaltens eine andere, nicht der ursprünglichen Faltung entsprechende Faltung, kann es vorkommen, dass der Ballon in der zweiten Stenose beim Rückziehen blockiert wird, da eben die ursprüngliche, den Hindurchtritt durch die Stenosen ermöglichende Faltung nicht mehr erreicht wurde.

Es ist daher Aufgabe der vorliegenden Erfindung, einen Katheter-Ballon der dem Oberbegriff des Anspruches 1 angegebenen Art zu schaffen, der ein besseres Einfalt-Verhalten als die im Stand der Technik bekannten Ballons ermöglicht.

Die Lösung dieser Aufgabe erfolgt durch die Merkmale des Anspruches 1.

Dadurch, dass beim erfindungsgemäßen Katheter-Ballon nur die verjüngten Endbereiche mit einer Form versehen sind, welche eine Rückfaltung unterstützen, ergibt sich ein besseres Einfalten beim Deflatieren, da im Gegensatz zum Stand der Technik, bei dem auch der Ballonkörper mit Faltungen oder Rippen versehen ist, beim erfindungsgemäßen Ballon keine Einflüsse durch vorgegebene Prägelinien auf den Ballonkörper ausgeübt werden, da dieser prägungsfrei ist.

Zum besseren Verständnis vorliegender Erfindung ist zu beachten, dass unter "Faltung" eines Katheter-Ballons ein Schritt nach der Ballonfertigung verstanden wird. Bei der Fertigung wird der Ballon bereits mit einer Form bzw. Prägung in den sich verjüngenden konischen Endbereichen gefertigt, welche unterschiedliche Wanddicken in dem jeweiligen Konusbereich erzeugen. Diese unterschiedlichen Wanddicken und Geometrien sollen das "Zurückfalten" in eine Mehrfachfaltung unterstützen. Dies wird beim erfindungsgemäßen Katheter-Ballon durch den Umstand perfektioniert, dass der Ballonkörper selber ohne eine vorgegebene Prägung ausgebildet ist.

Da beim erfindungsgemäßen Katheter-Ballon somit ausschließlich die sich verjüngenden bzw. konischen Endbereiche mit einer vorgegebenen Prägung versehen sind, induzieren Prägelinien dieser Prägungen die Faltung des Hauptkörpers, sodass das eingangs erwähnte verbesserte Faltverhalten erreicht wird.

Unteranspruch 2 hat eine vorteilhafte Weiterbildung der Erfindung zum Inhalt.

Durch den Umstand, dass die Faltung an einem Endbereich in Draufsicht auf diesen gesehen, nicht kongruent zu der am anderen Endbereich vorgesehen Faltung ist, sondern in Umfangsrichtung zu dieser versetzt angeordnet ist, ergibt sich der Vorteil, dass sich der Ballonkörper in eine definierte Richtung faltet. Hierdurch wird eine Erhöhung der Wahrscheinlichkeit der Rückfaltung in einen definierten Zustand, der der ursprünglichen Faltung entspricht, erheblich erhöht. Denn durch das Vorsehen des Versatzes der Prägungen an den beiden Endbereichen ergibt sich eine leicht verdrehte Form der Prägefalten, so dass sich diese in der definierten und identischen Richtung zurückfalten, so dass der Ballon seine ursprüngliche Form und Dimension einnimmt, die ein Blockieren in einer Stenose verhindert.

Durch den zuvor erläuterten Versatz, der vom distalen zum proximalen Ende des Ballon-Körpers verläuft, wird zusätzlich eine leichte Drehung erzeugt, welche die Richtung, in welche die Falten des Katheter-Ballons beim Auftreten auf einen Widerstand ausweichen können, vorgeben.

Weitere Einzelheiten, Merkmale und Vorteile der Erfindung ergeben sich aus nachfolgender Beschreibung anhand der Zeichnung.

Es zeigt:
- Fig. 1: eine schematisch vereinfachte Vorderansicht des erfindungsgemäßen Katheter-Ballons,
- Fig. 2: eine der Fig. 1 entsprechende Darstellung einer Draufsicht,
- Fig. 3: eine Stirnansicht eines konisch sich verjüngenden Endbereiches,
- Fig. 4: eine Stirnansicht des gegenüber liegenden sich konisch verjüngenden Endbereichs, und
- Fig. 5+6: die den Fig. 1 und 2 entsprechenden perspektivischen Ansichten des erfindungsgemäßen Katheter-Ballons.

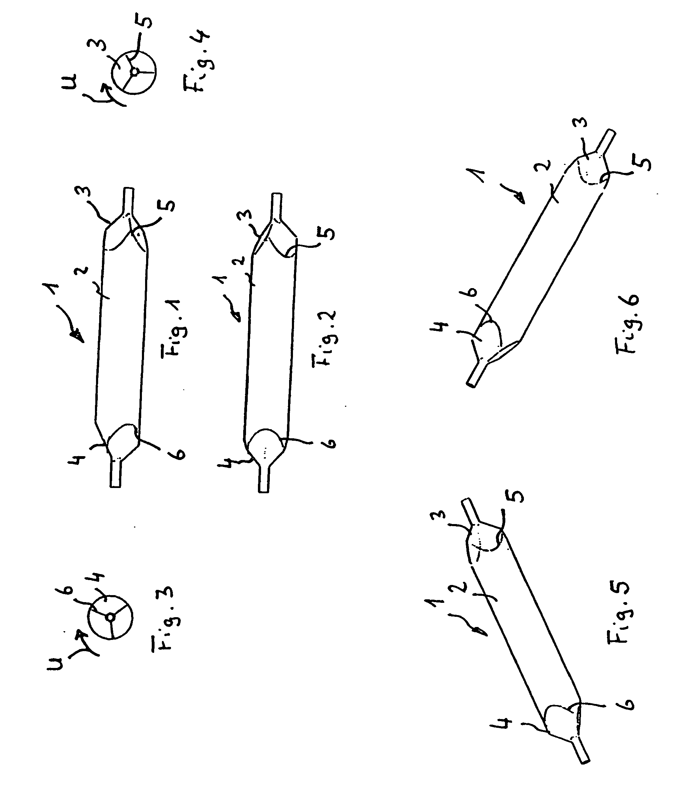

Die Fig. 1 und 2 verdeutlichen einen erfindungsgemäßen Katheter-Ballon 1, der einen im expandierten Zustand im Wesentlichen zylinderförmigen Ballonkörper 2 aufweist.

An beiden Enden des Ballonkörpers 2 schließen sich konisch verjüngte Endbereiche 3 und 4 an, die jeweils mit einer Prägung 5 bzw. 6 versehen sind.

Wie die Fig. 1, 2, 5 und 6 verdeutlichen, ist der Ballonkörper 2 selber prägungsfrei ausgebildet.

Die Fig. 3 und 4 zeigen, dass die Faltung 5 des Endbereiches 3 in Umfangsrichtung U gesehen, versetzt zur Prägung 6 des Endbereiches 4 vorgesehen ist. Hieraus ergibt sich der Vorteil, dass beim Einfalten die Prägungen 5 und 6 eine Faltung des Ballonkörpers 2 in definierter vorgegebener Richtung induzieren.

## Patentansprüche

1. Katheter-Ballon (1) mit einem im expandierten Zustand im Wesentlichen zylinderförmigen Ballonkörper (2) und mit zwei sich verjüngenden Endbereichen (3, 4) an den beiden Enden des Ballonkörpers (2), die jeweils mit einer vorgegebenen Prägung (5, 6) versehen sind, welche eine Rückfaltung unterstützen, wobei der Ballonkörper (2) ohne vorgegebene Prägung ausgebildet ist, **dadurch gekennzeichnet, dass** die Endbereiche (3, 4) abgeflachte Bereiche aufweisen, die von den Prägelinien der jeweiligen Prägung (5 bzw. 6) begrenzt sind.

2. Katheter-Ballon nach Anspruch 1, **dadurch gekennzeichnet, dass** die Prägung (5) in Draufsicht auf den einen Endbereich (3) gesehen, in Umfangsrichtung (U) des Ballonkörpers (2) versetzt zur Prägung (6) des gegenüber liegenden Endbereichs (4) vorgesehen ist.

## Claims

1. Catheter balloon (1) having a balloon body (2) which is substantially cylindrical when expanded and having two tapering end regions (3, 4) at the two ends of the balloon body (2), each of which is provided with predefined embossing (5, 6), which assist folding back, the balloon body (2) being formed without predefined embossing, **characterized in that** the end regions (3, 4) have flattened regions which are delimited by the embossed lines of the respective embossing (5 and 6, respectively).

2. Catheter balloon according to Claim 1, **characterized in that**, as seen in plan view of the one end region (3), the embossing (5) is provided so as to be offset in the circumferential direction (U) of the balloon body (2) with respect to the embossing (6) of the opposite end region (4).

## Revendications

1. Ballonnet de cathéter (1) avec un corps de ballonnet (2) sensiblement cylindrique à l'état gonflé et avec deux zones d'extrémité (3, 4), allant en se rétrécissant, présentes au niveau des deux extrémités du corps de ballonnet (2), qui présentent chacune une empreinte (5, 6) prédéterminée, qui soutien un pli arrière, le corps de ballonnet (2) étant réalisé sans empreinte prédéterminée, **caractérisé en ce que** les zones d'extrémité (3, 4) présentent des zones aplaties qui sont limitées par les lignes de l'empreinte respective (5 resp. 6).

2. Ballonnet de cathéter selon la revendication 1, **caractérisé en ce qu'**en vue de dessus en regardant la première zone d'extrémité (3), l'empreinte (5) est prévu décalée dans la direction circonférentielle (U) du corps de ballonnet (2) par rapport à l'empreinte (6) de la zone d'extrémité opposée (4).
